# EUROPEAN PATENT SPECIFICATION

(11) **EP 1 931 403 B1**
(45) Date of publication and mention of the grant of the patent: **04.01.2017**
(21) Application number: 06772018.5
(22) Date of filing: 02.06.2006
(51) Int. Cl.: A61M 1/00, A61M 1/10, A61M 1/12

(54) **AXIAL FLOW PUMP WITH MULTI-GROOVED ROTOR**
AXIALE FLUSSPUMPE MIT MEHRKERBIGEM ROTOR
POMPE AXIALE À ROTOR MULTI-RAINURES

(30) Priority: 05.10.2005 US 243722; 06.10.2005 WO PCT/US2005/035964; 22.11.2005 WO PCT/US2005/042495
(43) Date of publication of application: 18.06.2008
(73) Proprietor: HeartWare, Inc., Miramar, Florida 33025 (US)
(72) Inventor: LAROSE, Jeffrey, A., Parkland, FL 33076 (US); SHAMBAUGH, Charles, R. Jr., Coral Gables, FL 33134 (US); TRICHI, Kartikeyan, Miami Lakes, FL 33014 (US); MARQUIS, Richard, A., Miami, FL 33186 (US); WHITE, Daniel, G., Coral Springs, FL 33076 (US)
(74) Representative: Armstrong, Iain Cheshire
(86) International application number: PCT/US2006/021544
(87) International publication number: WO 2007/040663

(56) References cited:
- WO-A1-2005/032620
- WO-A2-2006/060260
- WO-A2-2006/118600
- US-A- 3 608 088
- US-A- 3 685 059
- US-A- 4 927 407
- US-A- 5 211 546
- US-A- 6 053 705
- US-A- 6 120 537
- US-A- 6 155 969
- US-B1- 6 227 797
- US-B2- 6 717 311

## Description

### FIELD OF THE INVENTION

The present disclosure relates to rotary pumps and, in particular, to axial flow blood pumps having a generally cylindrical rotor suspended within a corresponding cylindrical housing having a blood inlet at one end and blood outlet at another end, and motor components to provide rotational energy to spin the rotor and pump blood fluid longitudinally through the housing from the housing inlet to the housing outlet.

### BACKGROUND OF THE INVENTION

The known axial flow pumps (see e.g. WO 2005/032620) for blood have the advantage of narrow radial
width, when compared with centrifugal flow pumps. They may therefore be used for intra-vascular or intra-heart blood pumping assistance. Axial flow pumps typically have a cylindrical housing with an inlet at one end, an outlet at the opposite end, and a rotor within the housing which has thin impeller blades or vanes attached to and protruding radially outwardly from the rotor. Thus, as the rotor rotates, the blades add work to the fluid, propelling the fluid through the housing from the housing inlet to the housing outlet.

A suspension system is provided to maintain the rotor in a desired position within the housing, and an electromagnetic motor is provided to spin the rotor. The rotor may be mechanically, magnetically or hydrodynamically suspended within the blood flow passage. A combination of such suspension techniques may be utilized.

Typically in the prior art, the rotor is suspended by mechanical bearings or bushings, some with a rotor shaft protruding through the pump housing to a motor drive mechanism. Magnetic suspension is also known, as in U.S. Patent Nos. 6,368,083 and 5,840,070. The blood discharged from the pump, flows parallel to the axis of rotation of the rotor.

Axial blood flow pumps have heretofore used a thin blade design, with the motor magnets being placed either in the rotor shaft, relatively far away from the surrounding stator, as in pumps by Jarvik and Incor, or they use small magnets placed within the thin blades, as in a pump made by MicroMed. Both of these approaches tend to reduce the motor torque capacity and efficiency, and they require mechanical rotor support involving abutting surfaces that move and wear against each other in rotation.

It is desirable for blood pumps, whether internally or externally located, to be more tolerant of flow variations than the previous thin blade designs and to exhibit low hemolysis, good resistance to thrombosis, adequate system efficiency, and very high reliability for the expected duration of use for the device. Internally located blood pumps are also subject to anatomical compatibility design constraints and the need for elimination of mechanical wear and associated failure modes in order to provide successful, long-term, implantable devices.

While the pump of this disclosure is described in terms of a blood pump, it is also contemplated that the pump might be used for pumping chemically difficult fluids or non-magnetic fluids, where a sealless design is highly desirable, and the fluid must be gently handled for various reasons, for example because it is unstable to mechanical stress, causing decomposition and even explosiveness, or because it is another complex, biological fluid besides blood, having critical stability parameters.

### SUMMARY OF THE INVENTION

Where in the following the word invention is used and/ or features are presented as optional this should be interpreted in such a way that only protection is sought for the invention as claimed.

In accordance with the present invention an axial flow sealless and wearless blood pump is provided which comprises a tubular pump housing having a blood inlet at one open end and a blood outlet at the other open end opposite the inlet. A cylindrical rotor is suspended within the housing tube. The rotor comprises a plurality of peripheral and radial surfaces to engage and create pressure to assist in movement of the blood through the housing from the inlet end to the outlet end. A motor is provided to cause the rotor to spin within the housing. The motor stator includes electrically
conductive coils located external to or within the housing tube. A plurality of magnetic motor drive poles is provided on the rotor, spaced about its peripheral surfaces. The stator coil provides magnetic flux to cause the rotor to spin.

The rotor comprises a cylindrical body having a leading edge portion for engaging blood entering the housing at the inlet and a trailing edge portion for enhancing the discharge of the blood at the outlet of the housing. The rotor comprises one or more grooves each extending from an entry channel at the leading edge portion of the rotor to an exit channel at the trailing edge portion so as to define a plurality of arcuate peripheral land areas therebetween on the surface of the rotor. The sidewall surfaces defining each groove extend radially to the rotor surface but are not necessarily parallel to each other.

Each groove has a central portion defining a flow channel curved at least partially around the rotational axis of the rotor and in fluid flow communication with a substantially axially directed channel at the trailing edge portion of the rotor. The sidewalls of the grooves add axial thrust to the blood when the rotor is spinning and impart a rotational momentum to the flow of blood downstream of the rotor. In some embodiments, the central portion of each groove defines a narrower flow channel than is provided at its entry and exit channels. Each groove is wider at its
exit channel than at its entry channel to enhance the exit flow characteristics of the blood. In one embodiment, the combined total width of the central portions of the groove flow channels is substantially equal to or less than the collective, total arcuate widths of the peripheral land areas formed between the groove flow channels. The flow channels along the rotor may be helical along some portions of the rotor and generally axial directed along other portions of the rotor.

A plurality of hydrodynamic thrust bearing surfaces is provided on each of the peripheral surfaces of the land areas of the rotor. The bearing surfaces create fluid pressure at the periphery of the rotor thereby imparting radially symmetrical forces to the rotor, which maintain the radial position of the rotor within the housing when the rotor is spinning, and to provide good washing near the surrounding housing for increased resistance to thrombosis.

The land surface areas of the rotor between the flow channels of the grooves are each wider and longer at their peripheries than the thin blades of prior art axial flow blood pumps. This permits the emplacement or formation of relatively large motor drive magnets at or near the periphery of the rotor. Large drive magnets in the rotor increase magnetic force, and their placement at the rotor periphery reduces the gap between the magnetic poles of the rotor and magnetic flux generating coils of a motor stator. This arrangement improves motor torque capacity and electromagnetic efficiency of the pump. Axial magnetic stiffness provided by a motor of radial flux gap design may be used to assist in holding the rotor in its axial position within the housing.

A magnetic bearing system may be provided, as well as hydrodynamic thrust bearings, to help maintain the position of the rotor radially or axially within the tubular housing. Magnetic poles to assist in suspension of the rotor within the housing may be placed within the peripheral land surfaces between the grooves of the rotor to be attracted to or repelled by corresponding magnetic poles within or adjacent the surrounding pump housing.

In one embodiment, magnetic bearings may be used instead of hydrodynamic thrust bearings, to provide an all magnetic suspension system. Such magnetic bearings could be positioned or formed in the peripheral land areas of the rotor either forward or aft of the location of the motor drive magnets. Accordingly, a rotor in accordance with this invention does not require mechanical supporting structures upstream or downstream thereof. Hydrodynamic thrust bearings, with or without magnetic bearings, or exclusive magnetic bearings, will be sufficient to maintain the rotor in desired position during operation.

In some embodiments, the configuration of the tubular pump housing may include an annular sloped interior surface near the rotor's leading or trailing edge portions to provide a mechanical stop for axial movement of the rotor. Such a configuration provides additional axial support for the rotor, as may become necessary in the event of shock loading to ensure that the rotor remains in proper position within the housing. Alternately, a split housing configuration might be provided, with annular sloped surfaces at both the rotor leading and trailing edge portions, to provide radial support and axial support in both axial directions. The blood pump may also utilize one or more upstream and downstream flow straighteners or diffusers to enhance flow characteristics of blood as it enters or exits the pump.

A controller is provided to run the motor at a set rotational speed, which may be set, for example by the attending physician. Alternatively, the motor may be run at a rotational speed which varies in response to a physiological control algorithm.

Unlike axial flow pump designs heretofore using radial thin blade impellers, upstream and downstream struts or stator elements which may serve as flow straighteners or diffusers may be useful but are not required. The absence of these upstream and downstream flow straighteners permits a simpler mechanical design, with fewer axial tolerance concerns associated with their placement. Moreover, the absence of upstream flow straighteners or diffusers permits a pre-swirl to the upstream blood flow pattern that may serve to improve resistance to thrombosis.

In some embodiments, a volute may be used at the output end of the housing to improve the output flow characteristics of the blood. For example, a volute may be used to redirect the blood flow in a direction normal to the rotational axis of the pump. A volute may improve the output blood flow characteristics of an axial flow pump by converting rotational kinetic energy in the output flow from the axial flow pump to a slower output velocity having sufficient pressure for discharge into the vascular system.

The blood pump of this invention might be implanted within the vascular system or located within the chest cavity of a patient, such as the pericardial space, abdomen, or subcutaneously near the skin, in a manner similar to pacemaker implantation. Likewise, the pump may be kept external to the body for shorter term vascular circulatory support. Also multi-rotor or ganged rotor pumps having a plurality of axially aligned axial flow pumps of the type described herein could be used to provide single or bi-ventricular support, or even total circulation for the patient in the manner of a full, artificial heart. Moreover, such multi-stage pumps can be constructed with smaller diameter tubular housing for intra-vascular implantation.

### BRIEF DESCRIPTION OF THE DRAWINGS

A more complete appreciation of the present invention and many of the attendant advantages thereof will be better understood by reference to the following detailed description when considered in connection with the accompanying drawings, wherein:
FIG. 1 is a longitudinal sectional view of an implantable, sealless, axial rotary blood pump in accordance with this invention.
FIG. 2 is an elevational side view of a rotor of the rotary pump of Fig. 1.
FIGS. 3 and 4 are elevational views of two different sides of the rotor of Fig. 2.
FIG. 5 is a sectional view taken along line 5-5 of Fig. 2, with internal parts omitted.
FIG. 6 is a perspective view of an alternative embodiment of a rotor usable in the pump of this invention.
FIG. 7 is a rear perspective view of a rotor of the embodiment of Fig. 1.
FIG. 8 is a top perspective view of the rotor of Fig. 7.
FIG. 8A is an enlarged, fragmentary, perspective view of a portion of the rotor of Fig. 7.
FIG. 8B is an exploded view of an embodiment of the rotor of Fig. 7.
FIG. 9 is a longitudinal sectional view of an alternate embodiment of the pump of Fig. 1.
FIG. 10 is a plan view, taken partially in longitudinal section, showing a multiple-rotor blood pump of the present invention.
FIG. 10A is a plan view of another embodiment of the blood pump of Fig. 10.
FIG. 11 is an exploded view of an alternate embodiment of the axial flow blood pump of the present invention.
FIG. 11A is a perspective view of a motor stator of the embodiment of Fig. 11.
FIG. 12 is a schematic sectional view of a blood pump with a volute according to an embodiment of the present invention.
FIG. 13 is a perspective view of the blood pump with a volute shown in Fig. 12.
FIG. 14 is an exploded view of the blood pump with a volute shown in Fig. 12.
FIG. 15 is a perspective view of the interior of a volute according to one embodiment of the present invention.
FIG. 16 is a top plan view of the interior of the volute shown in Fig. 15.
FIG. 17 is a perspective view of the interior of a volute according to another embodiment of the present invention.
FIG. 18 is a perspective view of the interior of a volute according to a further embodiment of the present invention.
FIG. 19 is a perspective view of the interior of a volute according to yet another embodiment of the present invention.
FIG. 20 is a perspective view of a downstream flow straightener according to still another embodiment of the present invention.
FIG. 20A is bottom elevation view of the flow straightener shown in Fig. 20.
FIG. 21 is a perspective view of a downstream flow straightener according to a still further embodiment of the present invention.
FIG. 22 is a perspective view of a downstream flow straightener according to a yet further embodiment of the present invention.
FIG. 22A is a bottom elevational view of the flow straightener shown in Fig. 22.
FIG. 22B is side elevation view of the flow straightener shown in Fig. 22.
FIG. 23 is a perspective view of a downstream flow straightener according to yet another embodiment of the present invention.
FIG. 23A is a bottom plan view of the flow straightener shown in Fig. 21A.
FIG. 24 is a perspective view of an artificial heart utilizing axial flow rotary pumps of the type shown and described herein.

### DETAILED DESCRIPTION OF THE PREFERRED EMBODIMENTS

In describing the preferred embodiments of the present disclosure illustrated in the drawings, specific terminology is employed for sake of clarity. However, the present disclosure is not intended to be limited to the specific terminology so selected, and it is to be understood that each specific element includes all technical equivalents which operate in a similar manner.

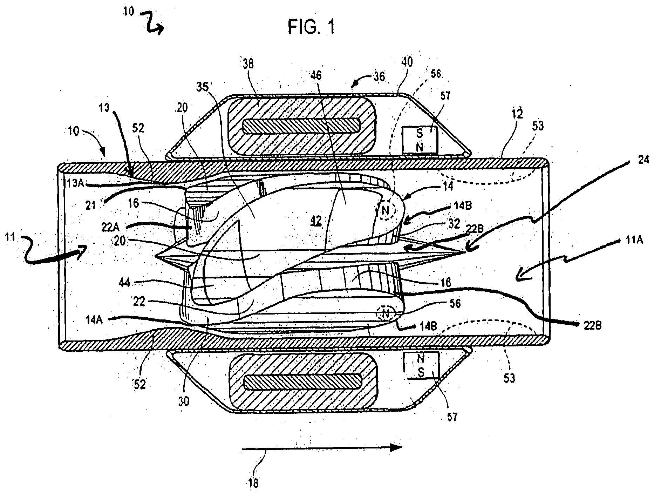

Referring now to the drawings and in particular to Figs. 1-5, an embodiment of a blood pump 10 adapted to assist in pumping blood through a patient's vascular system is disclosed, comprising a hollow generally tubular pump housing 12. The pump housing 12 is non-magnetic and is made of a suitable biocompatible material such as titanium or a suitable ceramic material which is non-thrombogenic, rigid and exhibits minimum eddy current lossess. The housing 12 defines a blood inlet end 11 and a blood outlet end 11A so that blood flows through the housing in the direction shown by the arrow 18. In one embodiment the housing 12 has a constant exterior diameter while the inlet portion of its interior diameter first converges as indicated at 13 and thereafter diverges as at 13A to define an annular hump or ring, indicated in Fig. 1 by reference numeral 52.

A substantially cylindrical rotor 14 is positioned within the lumen of the pump housing 12, and acts as an impeller for pumping fluid within the housing. In one embodiment, the rotor 14 is provided with a tapered leading edge 14A which is contoured to follow the diverging portion 13A of the interior diameter of the housing. The converging and diverging diameter portions 13 and 13A may act as a mechanical stop to maintain the rotor 14 in proper axial position within the tubular housing if, for example, an external shock would tend to jolt the rotor out of its working axial position. In some embodiments the tapered leading edge 14A of the rotor may be provided with a hydrodynamic thrust bearing surface of the type described below to cooperate with the surface of the diverging diameter portion 13A of the housing 12 for additional protection against axial shock loading. The alignment between the housing diverging diameter portion 13A and the tapered leading edge 14A of the rotor could also be utilized to provide a magnetic axial preload at the rotor's leading edge, similar to that described below with respect to its trailing edge, to assist the rotor in maintaining its suspended and wearless position within the housing.

Rotor 14 comprises one or more grooves 22 each of which extends from an entry section or inlet channel 22A at the leading edge 14A to an exit section or outlet channel 22B at the trailing edge 14B of the rotor. The grooves 22 define fluid flow channels across the rotor. In some embodiments a plurality of grooves 22 formed in the rotor 14 are spaced apart and define a plurality of peripheral land areas 35 therebetween. Each groove is defined by a pair of side walls 16 extending substantially radially to the rotational axis of the rotor, but not necessarily parallel to each other.

As shown in Figs. 1-4 and 6, each of the grooves 22 has a central flow channel 30 that curves at least partially around the rotational axis of the rotor and opens into a substantially axially extending outlet channel 22B. The curved central portion 30 is narrower than the inlet channel 22A or outlet channel 22B. The relatively wide outlet channel and its axial orientation enhances the discharge flow characteristics of the blood being pumped by more easily allowing for the release of blood from the rotor. The grooves 22 and their side walls 16 tend to drive blood in the axial direction, shown by the arrow 18, as the rotor 14 is rotated (clockwise in the embodiment of Fig. 1).

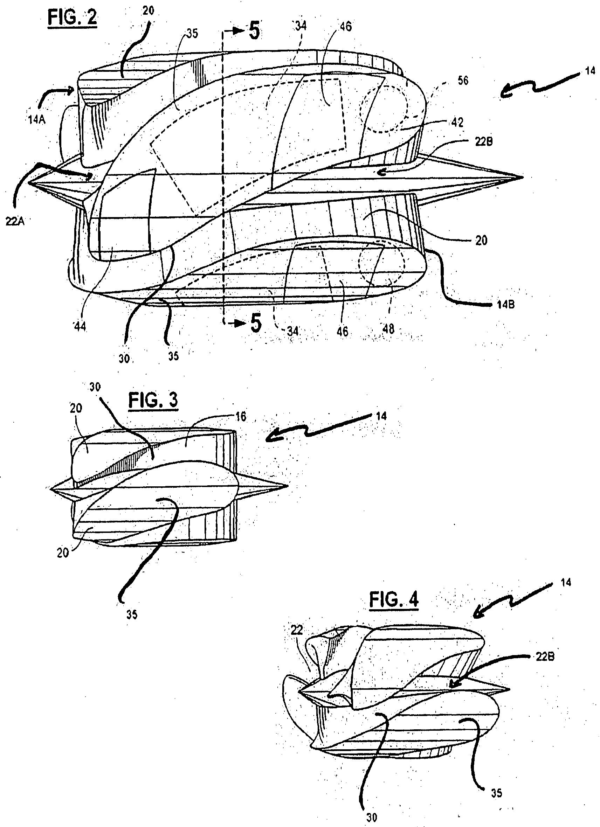

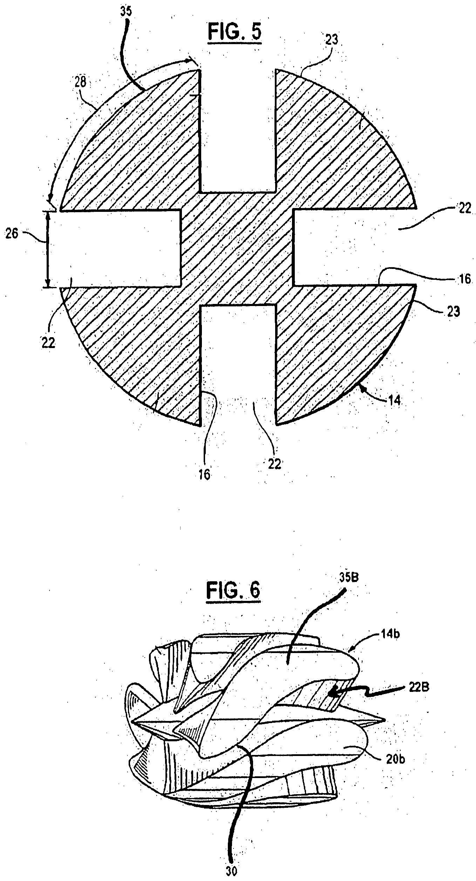

In one embodiment, the number of grooves 22 may be in the range of from 2 to 8, with four being typical. Irrespective of the number of grooves, their collective widths at the outer periphery 23 of the rotor 14 (Fig. 5) is equal to or substantially less than the collective, total circumferential width at the same outer periphery 23 of all of the land areas 35 defined between the grooves. By way of example, as shown in the embodiment of Fig. 5, the peripheral width of a groove 22 at the cross section of the rotor taken along the line 5-5 of Fig. 2 is shown by the arrow 26. The arrow 26 is shorter than the width of an adjacent land section 35 as measured by the length of the arc 28. Collectively, at the central portions along the grooves 22, the total width of the grooves 22 is less than or equal to the collective, total width of the respective land areas 35.

In this embodiment, the depth of each of the grooves 22 is greater than the radial extent of the blades in comparable and conventional thin blade axial pump designs. For example, for heart pump uses, the average depth of the grooves 22 from their outer perimeters may fall within the range of from 1mm to 5mm. In some embodiments the average depth of the grooves is approximately 1/3 the diameter of the rotor, but is less than the radius of the rotor. In other embodiments the grooves may be deeper at the entry channel 22A at the leading edge of the rotor and shallower at the exit channel 22B at the trailing edge of the rotor.

With reference to Fig. 2, the blood pump 10 further comprises a rotor, which includes a plurality of relatively large permanent drive magnets 34 (shown in dotted lines) formed within each of the wide land areas 35 of the rotor 14. According to one embodiment of the present invention, the permanent drive magnets 34 in the rotor may be produced by magnetizing selected portions of the peripheries of the land areas 35. This may be accomplished, for example, by constructing the rotor from a magnetic alloy, which may be isotropic, and magnetizing desired peripheral sections to form a plurality of magnetic poles with various geometric orientations. It is preferable to use a magnetic alloy that is biocompatible so that no additional coating is required. Such a rotor may be easier and less expensive to manufacture than impellers formed from multiple parts.

With reference to Fig. 1, the motor also comprises a motor stator 36 having electrically conductive coils 38. The coils are placed within an enclosure 40 which surrounds the tubular housing 12 and the rotor 14. The motor stator 36 serves to rotate rotor 14 by the conventional application of electric power to the coils 38 to create magnetic flux. The permanent drive magnets incorporated into the wide land areas 35 of the rotor are selected for magnetic properties, length, and cross-sectional area in order to provide good electromagnetic coupling with the magnetic flux created by the motor stator. Because of the relatively large surface area of the land areas, the nature and placement of the rotor magnets becomes relatively easy to effect. This arrangement provides strong electromagnetic coupling and the necessary magnetic axial stiffness to maintain the rotor in position. In one embodiment, the magnetic coupling between the stator flux and the drive magnets in the rotor creates torque, causing the rotor 14 to rotate clockwise. It will be understood by those skilled in the art that the rotor could be caused to rotate in a counterclockwise direction without departing from the scope of the invention.

The motor may be a three phase, brushless DC motor. In one embodiment the motor could be a toroidal, three phase and wye connected design. The stator may have a back iron design which is consistent with a typical radial flux gap motor. If desired, the motor stator can comprise a separate, hermetically sealed enclosure 40 that slides over the tubular housing 12 into position. A braised weld ring to the enclosure 40 outer surface may be used to secure the motor stator housing in position. Laser welding is one possibility for securing the motor stator enclosure 40 to the housing and obtaining a hermetic seal. The specific technology for accomplishing this known in the prior art.

Referring to Fig. 6, another embodiment of a rotor 14b for the blood pump of this invention is disclosed. Rotor 14b is shown to have six peripheral land sections 35b between the flow channels 22 having central portions 30. Otherwise, the nature and configuration of the rotor 14b is similar to the rotor of the other embodiments disclosed herein.

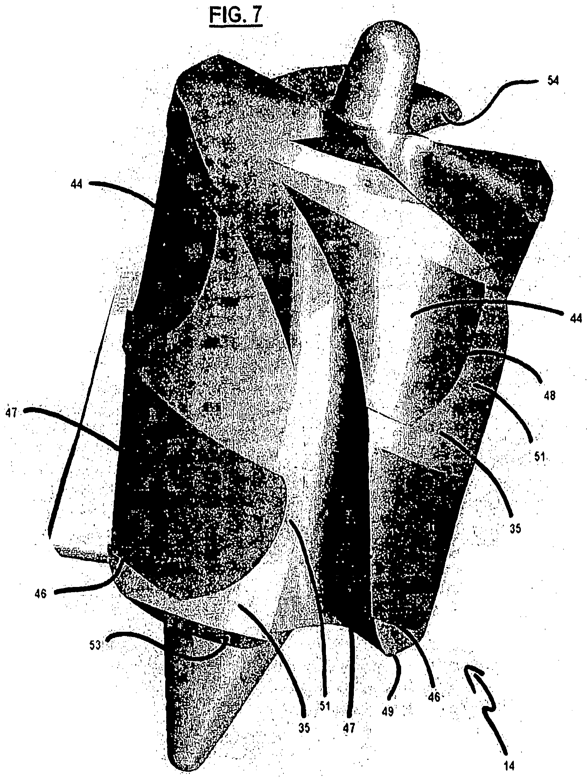

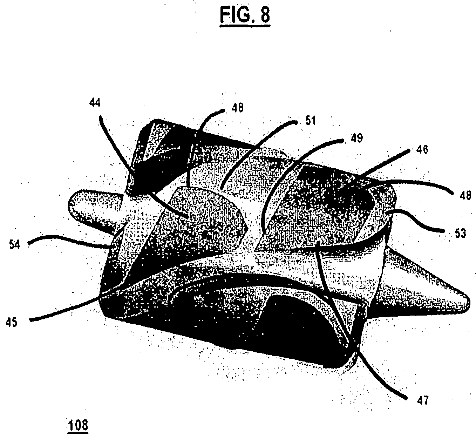

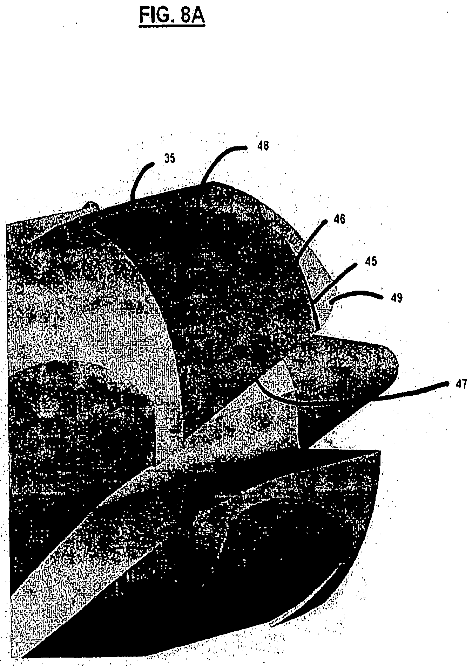

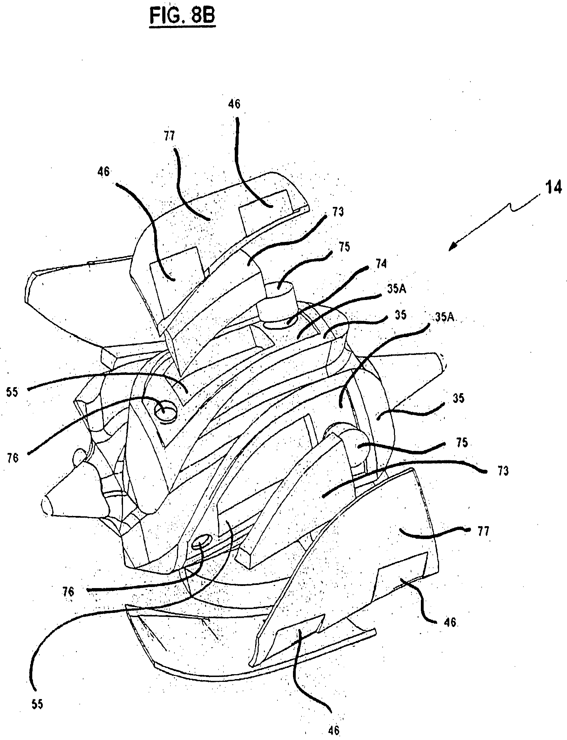

Referring to Figs. 7, 8, and 8A, there is depicted a rotor 14 which is similar to the rotor shown in the embodiment of Figs. 1-5. The peripheral land areas 35 of the rotor 14 are each provided with one or more hydrodynamic thrust bearing surfaces 44 and 46. Each of the thrust bearing surfaces 44, 46 is disposed along the surface of the associated land area having a prescribed peripheral radius. The leading edge 47 of each of the bearing surfaces from the viewpoint of the (clockwise) spin of the rotor 14, is recessed by a predetermined amount below the surface of the associated land section, as depicted in Figs. 8 and 8A by reference numeral 45. The recessed surface then tapers in a gradual, curved manner across the land area along an arc, the axis of curvature of which is not necessarily co-axial with the rotational axis of the rotor. The tapered bearing surface terminates at a rear end 48, at which point each bearing surface 44, 46 is feathered into the periphery of the land area with a smooth transition and is no longer recessed with respect to the continuing downstream surface of the land area.

As the rotor rotates, the respective thrust bearings, 44, 46 on each land area 35 scoop blood onto the bearing surfaces whereby it flows between the bearing surfaces and the inner wall of the tubular pump housing. The effect of the tapered configuration of the thrust bearing surfaces is to force blood to flow through a decreasing or constricting area created between the bearing surfaces and the inner wall of the tubular pump housing. This results in increasing fluid pressure upstream within the constriction, which pressure acts against the bearing surface areas and produces a net symmetrical force for radial support of the spinning rotor. That hydrodynamic thrust bearings act in this way to cause radial pressure on a rotor is well known to the art generally, as in U.S. Patent No. 5,840,070. The hydrodynamic force that is thus created on the surfaces of the rotor land areas tends to hold the rotor suspended and centered within the lumen of the tubular housing 12 in a manner shown in Fig. 1, and resists dynamic, radial shock loading forces without the need for physically contacting bearing surfaces. The thrust bearing surfaces 44 and 46 may be formed directly into the peripheral surfaces of the land areas 35 or may be placed within suitable cavities formed in the outer peripheral surfaces of the land areas and held in place by a suitable cover.

In some embodiments, hydrodynamic thrust bearing surfaces are created on the leading or trailing edge portions of the rotor. For example, with reference to Figs. 1-3, the surface area 20 at the leading edge 14A of the rotor is tapered into a suitable thrust bearing configuration to cooperate with the diverging interior surface 13A of the tubular pump housing. Such a thrust bearing would resist longitudinal movement of the rotor to the left, as shown in Fig. 1. Alternatively, the diverging portions 13A partially defining the annular ring 52 may, if desired, comprise hydrodynamic thrust bearings cooperating with the adjacent rotor surface to prevent contact between the rotor 14 and the ring 52 as the rotor operates in a clockwise rotation.

Hydrodynamic thrust bearing surfaces may also be located on the rotor near its trailing edge 14B, in which event the inner diameter of the tubular pump housing near its outlet end 11A would be constricted as shown in dotted lines in Fig. 1 to define an annular ring 53 similar to the ring 52 near the inlet end 11. Such thrust bearings on the rotor or formed on a side of the ring 53 would serve the similar purpose of replacing or of supplementing the repulsive magnetic poles of magnets 56 and 57 described below. Such thrust bearings may provide one or both of radial and axial support for the rotor and serve to increase the resistance to shock loading thereby improving rotor stability.

Hydrodynamic thrust bearings on the outer periphery of the rotor provide good surface washing. Centrifugal forces created by thrust bearings tend to push fluid toward the periphery of the housing interior, providing increased blood flow, which can improve the pump's resistance to thrombosis. In contrast, hydrodynamic bearings in the prior art which are closer to the axis of rotation have reduced surface washing, resulting in a greater possibility of blood coagulation. Thus, since by this invention, conditions are provided that reduce blood coagulation, a lower amount of anticoagulant may be used with the blood pump and patient, which may result in fewer patient adverse side effects. If desired, hydrodynamic thrust bearing surfaces may be aligned in a helical fashion on the surfaces of the rotor to improve surface washing by the moving blood as the rotor spins.

As an alternative to hydrodynamic thrust bearings acting axially on the rotor, permanent rotor retaining magnets maybe placed in each land area 35 within the lead, trailing or both ends of the rotor. One or more corresponding permanent magnets may be placed within or on the tubular pump housing adjacent each rotor retaining magnet to effect repulsive magnetic forces acting to retain the axial alignment of the rotor within the housing. By way of example only, a permanent magnet 56 is shown in Figs. 1 and 2 in dotted lines on a land surface area at the trailing end of the rotor 14. A corresponding permanent stator magnet 57 is emplaced within the enclosure 40 surrounding the tubular housing 12. The rotor magnet 56 may be formed by magnetizing suitable rotor material. If the north poles of the rotor magnet 56 and the stator magnet 57 are adjacent or face each other, as shown in Fig. 1, the repelling magnetic forces will assist in retaining the rotor in the proper axial position. Longitudinal or axial movement of the rotor to the right is thereby restricted by the repulsive action of magnets 56 and 57. Of course, magnetic south poles could be directed to face each other in similar manner, to achieve a generally similar effect. It will be understood that the magnet 57 may comprise a ring magnet or an electromagnetic coil.

With reference to Fig. 8A, in one embodiment, each of the thrust bearing surfaces is provided with shrouds 49 provided along each lateral side of a thrust bearing surface. These shrouds, defined by sidewalls of decreasing height created by the recessed portion of each bearing surface, reduce the amount of fluid leakage from the bearing surface, and allow the development of higher radial pressure levels. The reduction of such leakage to acceptable levels by means of such shrouds can almost double the load carrying capacity for the bearings.

An optional pressure relief surface downstream of each rotor thrust bearing surface may be provided to reduce hemolysis. This pressure relief surface consists of a portion of the peripheral land area that is contiguous with the rear end 48 of a thrust bearing surface and slightly diverges away from the housing wall. Blood passing over the thrust bearing surface is thereby directed across the pressure relief surface into an adjacent one of the grooves 22 formed in the rotor. Rounded surfaces 54 at the leading end of the rotor, seen in Figs. 7 and 8, facilitate entry of blood into the flow channels of the rotor. Thus, an axial flow pump having wide peripheral land areas and utilizing shrouded hydrodynamic thrust bearings for radial or axial support is provided, having significant advantages over the known types of axial flow blood pumps.

In some embodiments, the rotor 14 may be produced by either machining, molding, or casting a single piece of ferromagnetic material, such as compression bonded neodymium or Alnico (aluminum-nickel alloy), or an alloy of about 70-80 percent by weight of platinum and about 20-30 percent by weight of cobalt. In some embodiments, from essentially 76-79 percent by weight of platinum is present in the alloy. In some embodiments, the alloy may contain essentially from 21-24 percent by weight of cobalt. In one embodiment, an integral, one-piece rotor consists of essentially 77.6 percent by weight of platinum and 22.4 percent by weight of cobalt. Such a rotor is conventionally heat treated to achieve good magnetic properties, and may be magnetized, with North and South magnetic poles, as desired.

An advantage of such a rotor is that a single, integral piece made from the platinum and cobalt alloy can be easily fabricated into complex shapes, using conventional metal working and casting methods. Also, such an alloy is magnetically isotropic, so that parts can be easily magnetized with a plurality of magnetic poles in any geometric orientation. These characteristics allow the rotor to be fabricated from a solid piece of the alloy, thus eliminating the need to build assemblies of magnets and support structures, as in the case of prior art ventricular assistance devices, with a resulting reduction of manufacturing costs. Additionally, the alloy used in this invention is biocompatible, and has high resistance to corrosion, also having a Rockwell hardness on the order of 31 Rc, which eliminates the need for a hard, outer coating. It will be understood that the rotor material may be isotropic or anisotropic, as desired.

After fabrication, the rotor may be treated with a conformal, protective polymer coating of an organic polymer such as Parylene, or silicone, to prevent against oxidation by forming a hermetic seal around the rotor. On top of this, a hard, lubricious protective coating may be applied over the conformal polymer coating, to protect against wear and abrasion. Such coatings may include chromium nitride, titanium-nitride, or other commercially available coatings such as ME92, Med Co 2000, or DLC. Alternatively, as stated above, the use of a biocompatible magnetically isotropic alloy such as a platinum-cobalt alloy obviates the use of the protective coating. Designed for a permanent heart ventricular assist device, such a rotor could be a cylindrical device having a 10 millimeters outer diameter and 20 millimeters length, providing flow rates of 2-10 liters per minute against physiologic, differential blood pressures. Magnetization of the rotor land sections may occur before or after a coating application.

With reference to Fig. 8B, an embodiment of the rotor 14 includes recesses 35A formed in each of the land areas 35, each of which recesses contains a cavity 55. Each cavity 55 is adapted to receive a discrete permanent drive magnet 73. The permanent drive magnets 73 serve the same purpose as the magnetized areas 34 described above in connection with Fig. 2. In this embodiment, the land area recesses 35A include cavities 74 adjacent each of the cavities 55. The cavities 74 are adapted to receive discrete permanent retaining magnets 75 which serve the function of the rotor retaining magnets 56 described above in connection with Figs. 1 and 2. The land area recesses 35A also include bores 76 formed for the purpose of weight reduction and to achieve dynamic rotational balance in the rotor when desired. A contoured cover 77 is adapted to be inserted into each of the land area recesses 35A to retain the discrete drive magnets 73 and retaining magnets 75 in position on the rotor. In this embodiment the covers 77 contain the hydrodynamic thrust bearing surfaces 44 and 46 for the rotor described above in connection with Figs. 7, 8 and 8A.

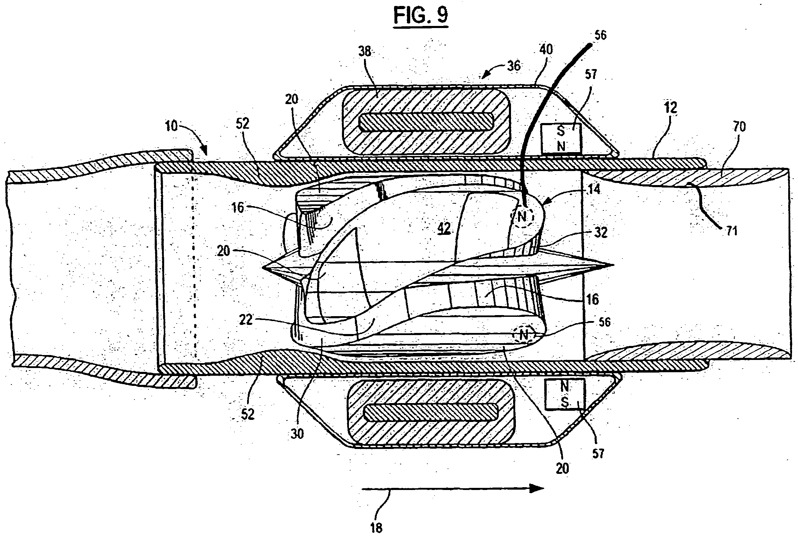

Referring to Fig. 9, there is disclosed an embodiment of the pump of Fig. 1 having a sleeve 70 inserted within the outlet of the housing 12 and having a reduced internal diameter area 71. The reduced internal diameter area 71 serves as a stop to mechanically retain the rotor 14 against movement in one axial direction, to the right in Fig. 9, so that magnets 56 and 57 may be unnecessary. In addition, the reduced internal diameter configuration of the sleeve 70 renders this arrangement suitable as a pediatric version of the axial flow pump of this invention as it will result in a reduced flow rate compared to an unsleaved configuration.

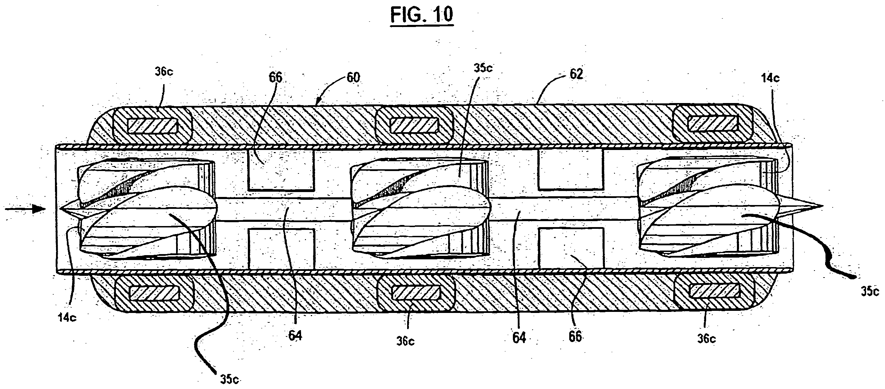

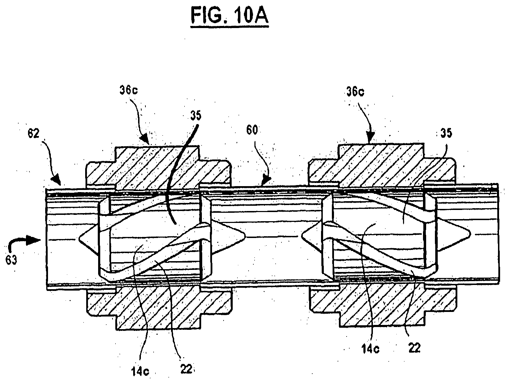

Referring to Fig. 10, a ganged series of axial flow blood pumps 60 has a common, cylindrical housing 62 in which a plurality of rotors 14c are mounted on a common shaft 64 in spaced-apart axial relationship. In one such embodiment, the rotors are commonly driven by the shaft 64 to rotate as one. Such a device is described in co-pending application Serial No. 11/118,55 1.

Each of the rotors 14c has peripheral land areas 35c, similar to the land areas 35 of the previous embodiments. By this means, added pumping power can be provided in the form of a multiple stage pump, with the rotors in series connection. Accordingly, a high capacity pump of smaller diameter can be provided.

Motor stators 36c, comprising electrically conductive coils are provided, one for each rotor, so that each of the respective rotors performs in a manner similar to that of the rotors described for previous embodiments, but for their connection with the common shaft. The rotors 14c and stators 36c may be of the same design as any of the previous embodiments, however, each rotor need not have the same number of grooves or land sections between the grooves.

Stator blades 66 of traditional thin blade design may be mounted to extend radially inwardly from the inner wall of pump housing 62 downstream of at least two of the three ganged rotors, although such blades are not normally required in the axial flow pumps of the present invention. The stator blades 66 serve to diminish the rotational momentum of the axial flow output from the rotors before the flow encounters the next rotor. This arrangement permits more hydraulic work to be added to the blood or other fluid. Any desired number of these generally radially extending blades 66 may be provided, if desired. Moreover, if desired, the leading or trailing end of each of the stator blades 66 may be provided with suitable hydrodynamic thrust bearing surfaces to provide additional axial support to the rotor. The stator blades 66 may also include integral permanent magnets to define magnetic bearings to support the rotor. Permanent magnets mounted in or on the appropriate leading or trailing ends of each rotor can provide repulsive magnet poles to assist in the axial stability of the rotors.

In the embodiment shown in Fig. 10, each of the motor stators 36c is axially aligned with its corresponding rotor. Such alignment may be altered to accommodate magnetic coupling or magnetic repulsion to provide extra axial magnetic support.

Referring to Fig. 10A an alternative multi-rotor axial pump consists of a plurality of blood pumps 60 each of which has one of the pump rotors 14c having the characteristics of the rotors 14 described above. The rotors 14c are axially aligned in spaced-apart relationship and adapted to pump blood or other fluid consecutively through the common cylindrical housing 62 made of biocompatible material that exhibits minimum eddy current losses, as described above in connection with single rotor pumps. In this embodiment, the individual rotors 14c function independently without a connecting shaft. Motor stators 36c each comprise an electrically conductive coil as in the previous embodiments, one for each rotor, so that the respective rotors perform in a manner similar to that of the previous embodiments. The stators 36c also may be of a design as previously described. The multiple rotors 14c acting in concert provide added pumping power and therefore enables a high capacity pump of smaller diameter than a single stage pump, and may be adapted for implant directly into the vascular system of a patient and otherwise reduce patent trauma.

In one embodiment, the independently rotatable rotors 14c rotate at the same rate. It will be understood that the rates of rotation of the multiple rotors may vary, one from the other, as desired. In some embodiments, one rotor may rotate clockwise, and be oriented such that its grooves tend to drive blood or other fluid through tubular housing 62 in the direction of arrow 63. An adjacent rotor may be oriented such that its grooves tend to drive blood in the same direction 63 upon counterclockwise rotation. Thus, the multiple rotors work together to drive fluid in direction 63, even while they rotate in opposite directions. An advantage of this arrangement is that a rotor rotating counterclockwise downstream from a clockwise rotating rotor tends to counteract the rotational momentum imparted to the pumped fluid by the upstream rotor. This permits more hydraulic work to be added to the fluid. Depending upon the power applied to the individual stators 36c, the respective rotors maybe driven at rotation rates which are similar, or different from each other, as may be desired. Adverse affects from misaligned motor drive waveforms are thereby reduced.

In some embodiments, the multi-rotor pump is free of stationary, swirl suppressing blades positioned within the housing and between the rotors. A need for such blades is diminished by counterrotating characteristics of the respective rotors.

In some embodiments, more than two rotors are present. Adjacent rotors will rotate in opposite directions from each other, so that clockwise rotating rotors are interspersed with counterclockwise rotating rotors in axially alignment within the pump housing.

A permanent ventricular assist device of multistage configuration as described above could have an outer diameter of six millimeters and a length of 15 millimeters, to provide flow rates of 2-8 liters per minute against physiological differential pressures, as previously described. Such a multi-stage pump could be used as a peripheral vessel blood insertion pump, operating outside of the body, or provide bi-ventricular support and even total artificial heart action. It will be understood, that the multiple rotors need not be ganged on a common shaft and that the motor stator for each rotor could be energized to effect clockwise or counterclockwise rotation of each rotor independently of the rotational spin of other adjacent rotors.

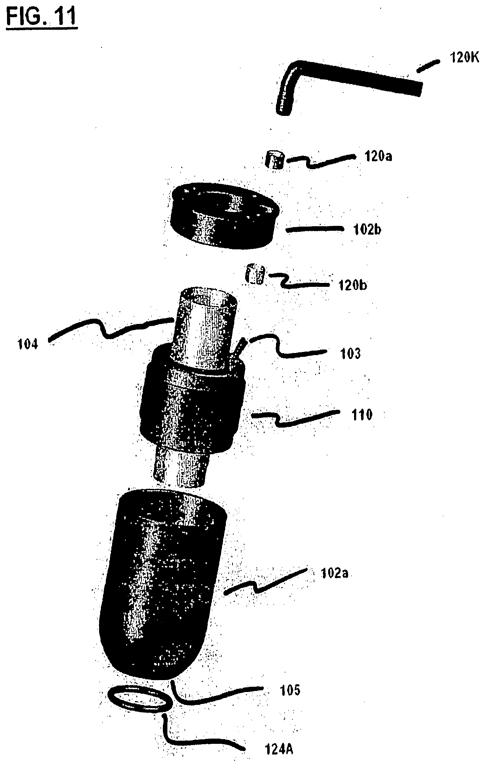

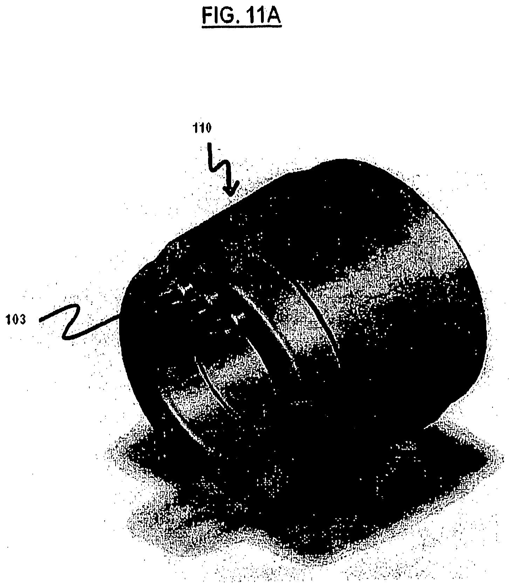

Fig. 11 is an exploded view of an alternative blood pump configuration according to an embodiment of the present invention. The pump may comprise a primary outer cannula-like enclosure 102a and a secondary or discharge section 102b that fit together to seal a tubular housing 104 and a surrounding motor stator 110 in place within the assembled enclosure. An O-ring 124A may be used to prevent blood from leaking between the inner tubular housing 104 and the enclosure 102a. In this embodiment, the entire tubular housing and surrounding motor stator are enclosed with the cannula-like structure have an inlet opening 105 of reduced diameter, which provides a bullet-like configuration.

The motor stator 110 has three electrical cables 103 (seen best in the enlarged view of the motor stator in Fig. 11A) for three phase operation of the motor coils. The electrical cables may be contained within a suitable cable conduit comprising the three sections 120k 120a and 120b. It will be understood that other motor designs may be selected for applications requiring high speed communication or increased efficiency, without departing from the scope of the invention.

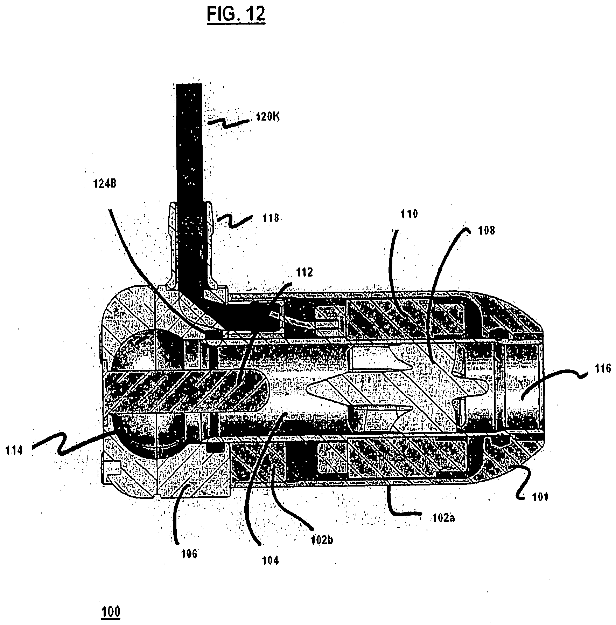

Figs. 12-16 depict an embodiment of a blood pump in which the rotational kinetic energy of the axial flow of blood discharged by an axial flow pump is converted into a pressure flow at the outlet of the pump by a volute, indicated by reference numeral 106. While the incorporation of a volute is not necessary with the axial flow pump of the present invention, it is an optional embodiment for improving blood flow characteristics to further minimize thrombus formation and increase pressure of the pumped blood as it enters the vascular system.

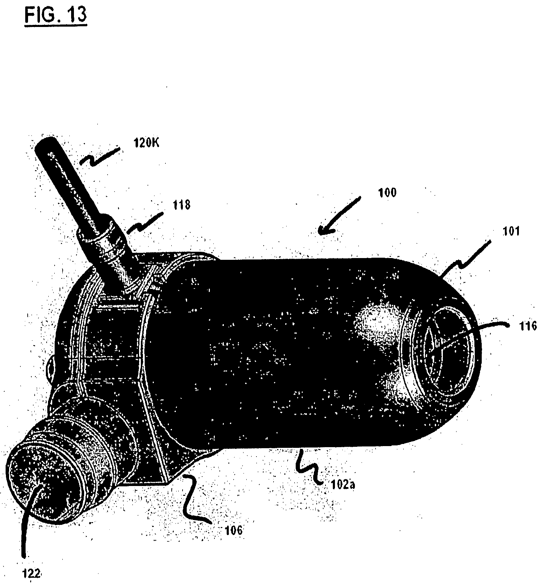

Referring to Fig. 12 and 13, a blood pump 100 comprises a substantially cylindrical outer enclosure or cannula 102a. The cannula 102a may have the slightly rounded or bullet shaped front or inlet end 105 of reduced diameter having inlet 116 through which blood enters the pumping chamber. The pumping chamber is defined by the substantially tubular interior housing 104 having an external diameter smaller than the internal diameter of the cannula. The cannula 102a and tubular housing 104, as described above, may be made of a biocompatible non-magnetic material such as titanium or ceramic.

The motor stator ring 110 may be located on the outside the housing 104 and within the cannula 102a in the annular space formed between the housing 104 and the cannula 102a. The three phase control wires for the coils of the stator ring 110, described in detail above, are connected through the power and control cable conduit 120k that exits the pump through a port 118 which may be defined as part of the volute 106. A rotor 108, of the type described in detail above, may be magnetically or hydrodynamically suspended in operation within the housing 104 and centered within the stator ring 110 to provide an axial flow of the blood or fluid entering the inlet 116.

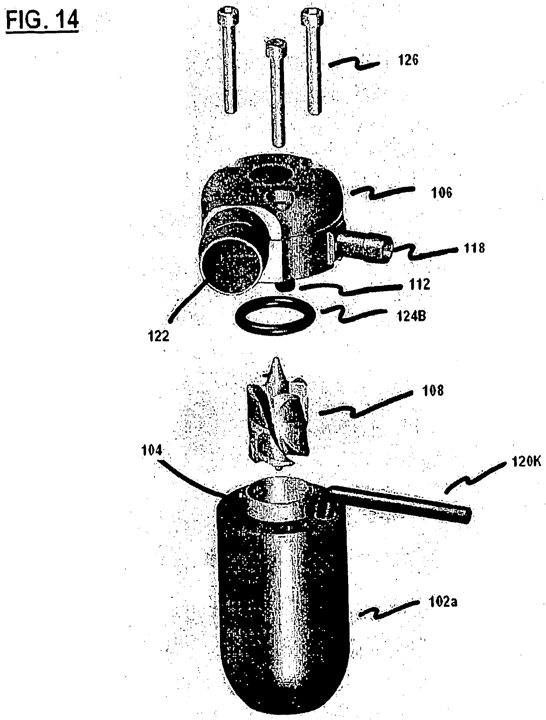

The volute 106 is sealed to the cannula 102a and the tubular housing 104 in a fluid-tight connection such that blood pumped by the rotor 108 is moved into a central chamber 114 (Fig. 12) of the volute 106. With reference to Figs. 12 and 14, an O-ring 124B may be used to ensure a fluid-tight connection of the volute to the inner tubular housing 104. One or more screws 126 may be used to secure a hermatic connection.

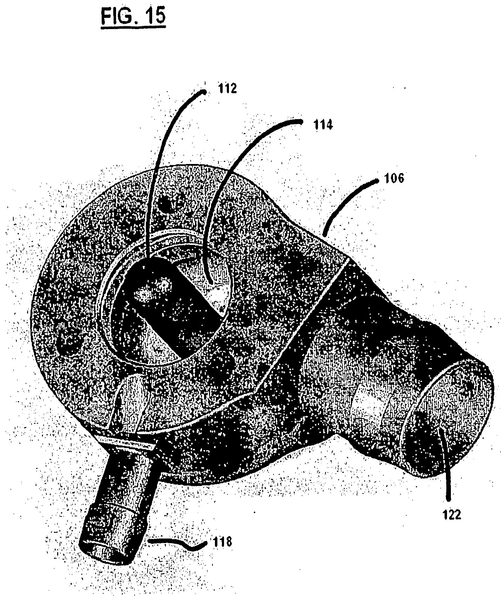

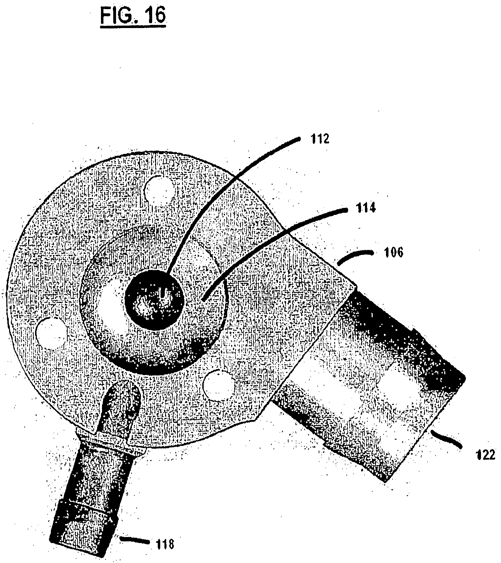

As depicted in Figs. 15 and 16, the volute chamber 114 may be annular in cross section as defined by a downstream center post 112 projecting inwardly along the pump axis from the base of the volute along the rotational axis of the pump rotor 108. The center post 112 extends toward but does not contact the downstream end of the rotor 108, and may be a dome-topped cylinder (as shown in Figs. 12, 14 and 15) or may be another shape that serves to affect the flow of blood discharged from the pump rotor, as described in detail below.

Blood driven by the rotor 108 and entering the volute chamber from the pump chamber of the axial flow pump has a rotational or spiraling momentum around the rotational axis of the rotor. The rotational momentum of the flow creates lower pressure areas in a central portion of the blood flow just downstream of the rotor. To some extent the lower pressure area is alleviated by a tapered axial extension 24 (Fig. 1) at the trailing edge 14B of the rotor. The center post 112 also tends to fill this lower pressure area in the downstream rotational blood flow characteristics as the blood enters the chamber 114 of the volute. Blood thereafter fills the annular chamber 114 of the volute and the fluid pressure of the system causes the blood stream to flow in a substantially centrifugal direction through the chamber 114 to the volute discharge or outlet 122, depicted in Figs. 13-16, thereby establishing the output pressure. In this embodiment, the volute is bladeless and the discharge blood flow is in accord with the longitudinal nature of the blood flow within the vascular system. Typically, a blood pump of this embodiment will be implanted such that the cannula portion traverses the apex of a heart ventricle, while the volute portion remains outside of the heart. A graft (not shown) is used to connect the discharge or outlet of the volute to an artery of the vascular system of the patient.

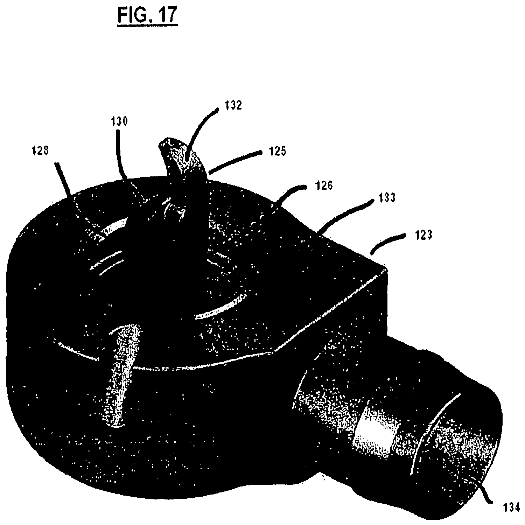

Referring now to Fig. 17, there is depicted an embodiment of a centrifugal volute 123 with an.alternate configuration for a flow straightner adapted to extend generally axially into the pump chamber of an associated axial flow pump. In this embodiment, the volute 123 has a flow chamber 133 of generally circular cross section from which extends a dual legged stator element 125 projecting out of the volute chamber and inwardly with respect to and along the axis of an axial pumping chamber as described above (not shown). The stator element has a pair of parallel legs 126 and 128 extending substantially co-axially with the rotational axis of the pump. The inner end portions of each of the supporting legs 126 and 128 are bent or curved such that the end portion 130 of the leg 128 is curved to project at an angle of about 45° to the longitudinal axis of the stator element 125 and the axis of an associated pumping chamber. The inner end portion 132 of the support leg 126 is also curved to project at angle of 45° to the pump axis. The axis of curvature of the end portion 130 is perpendicular to the axis of curvature of the end portion 132. The dual legged stator 125 acts to change the kinetic rotational momentum of the blood flow output from the axial pump to a pressure flow as the blood enters the centrifugal chamber 133 of the volute 123 before discharge through radial outlet 134.

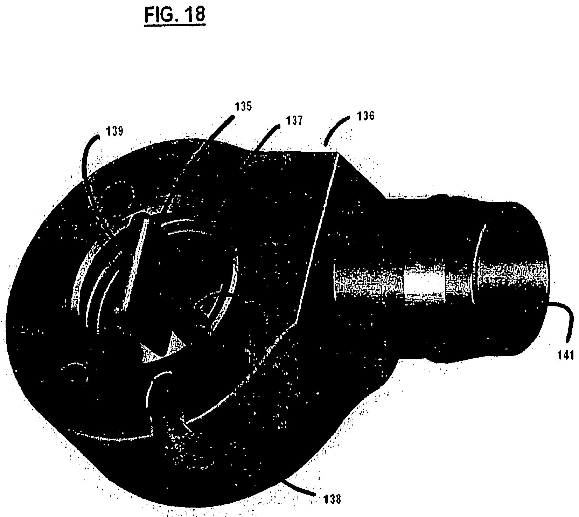

Referring to Fig. 18, there is shown still another embodiment of a volute 136 having a centrifugal flow chamber 137 of substantially circular cross section with a central axial flow straightener or stator element 135 adapted to extend generally axially into the pump chamber of an associated axial flow pump of the type described herein. The stator element 135 consists of a center post portion 138 aligned with the axis of the axial flow pump (not shown) having a tip or end portion 139 of generally rectangular shape. The short axis of the rectangular shaped stator tip is aligned and parallel with the axis of the center post portion 138 and the axis of the axial flow pump. The function of the stator element is, as described above in connection with the other volute embodiments, to alter the kinetic rotational momentum of the outflow from the pump to a pressure flow as the fluid fills the volute chamber and is pressured to discharge at the radial outlet 141.

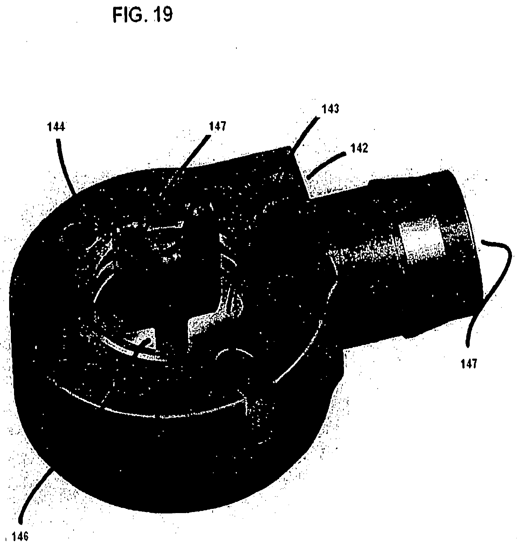

Referring to Fig. 19, there is depicted yet another embodiment of a centrifugal volute 142 having a centrifugal flow chamber 143 of circular cross section. A flow straightener or stator element 144 extends from the base of the volute chamber axially inwardly along and substantially aligned with the axis of an associated axial flow pump (not shown). The stator element 144 consists of a central post section 146 with a wide double-tined end portion 147. Each of the tines extends generally parallel to the axis of the center post 146, one on each side thereof. Blood exiting the axial flow pump with kinetic rotational momentum is converted to a pressure flow by the stator element 144 before entering the volute chamber and being forced centrifugally to discharge from an outlet 147.

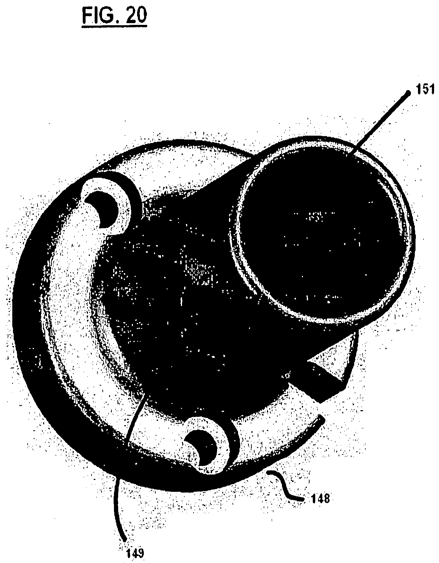

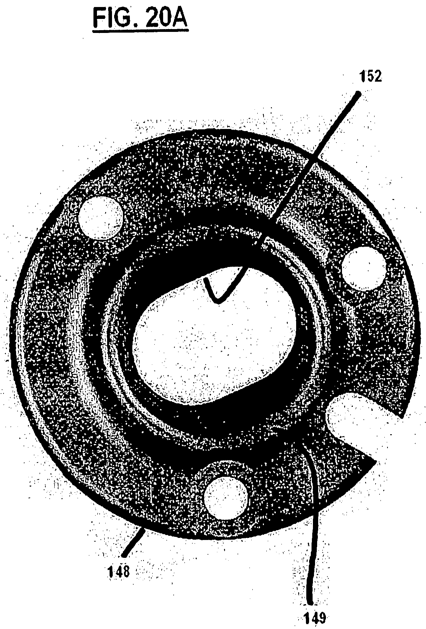

In accordance with the present invention, blood outflow characteristics may be altered without the need for projecting blades or posts downstream of the axial flow pump. Shaped passageways designed to improve flow characteristics may be employed instead. With reference to Figs. 20 and 20A, there is depicted a straight through flow straightener according to one embodiment of the present invention. The flow straightener has a base section 148 for securely connecting to the blood pump. A straight cylindrical tube 149 may extend from the base section 148. A passageway having a circular opening 151 may be shaped into an oval cross section with an oval outlet 152 formed within the tube 149 as shown in Fig. 20A. The opening of the shaped passageway 151 may be of any shape helping to enhance flow characteristics. The shaped passageway 161 defining the oval outlet 152 gradually ushers blood having rotational momentum through the oval-shaped constraint to convert the flow to substantially axial flow. Alternately, the shaped passageway 151 may be partially conical, having a circular, oval or other shaped outlet somewhat smaller in diameter than that of the inlet opening to accomplish the same purpose. In this embodiment, the passageway is 151 is straight and coaxial with the axis of the axial flow pump.

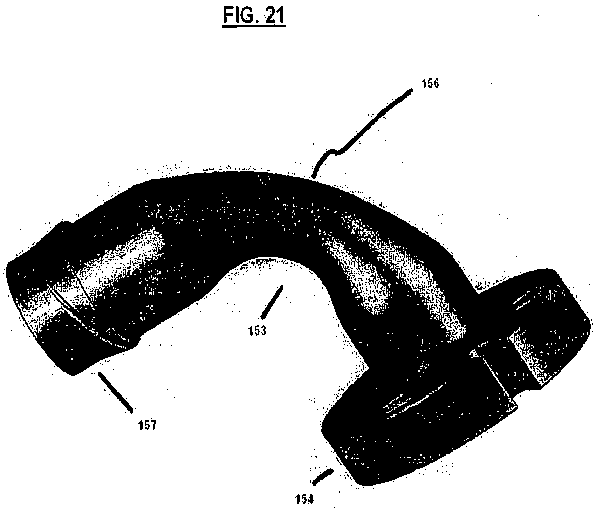

With reference to Fig. 21, there is depicted a flow straightener embodiment according to a yet further embodiment of the present invention. The flow straightener of this embodiment has a base section for securely connecting to the axial flow blood pump. A bent tube 153 contains a constricted portion 156 which acts to diminish the rotational momentum of the axial blood flow output from the pump. The axial blood flow from the tube 153 is discharged through an outlet 157 from which a suitable graft will connect the blood flow to the vascular system.

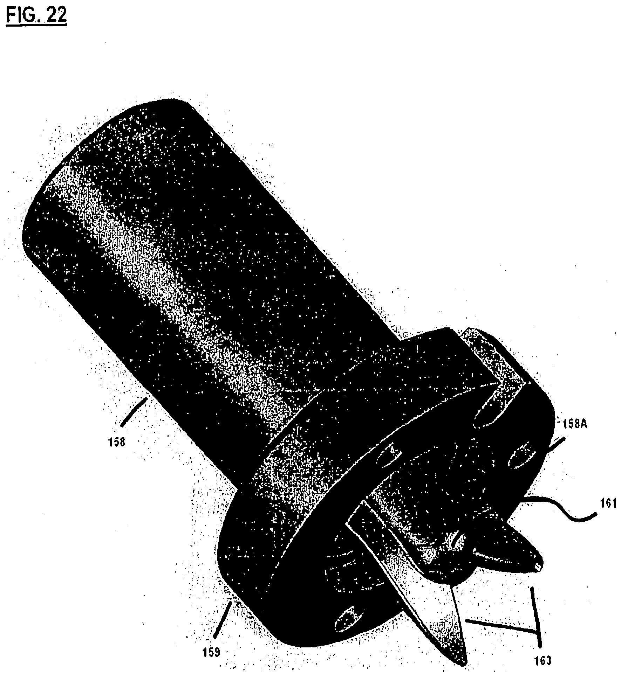

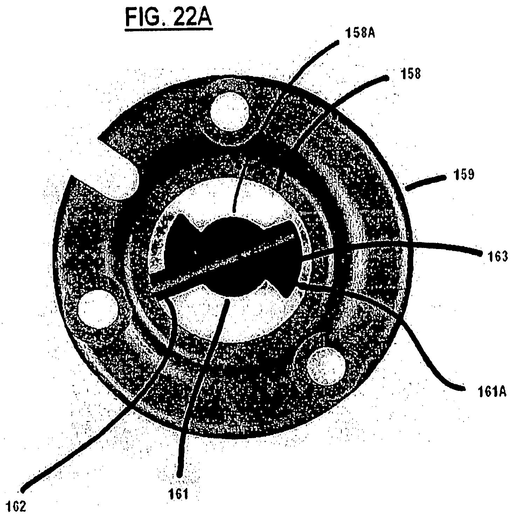

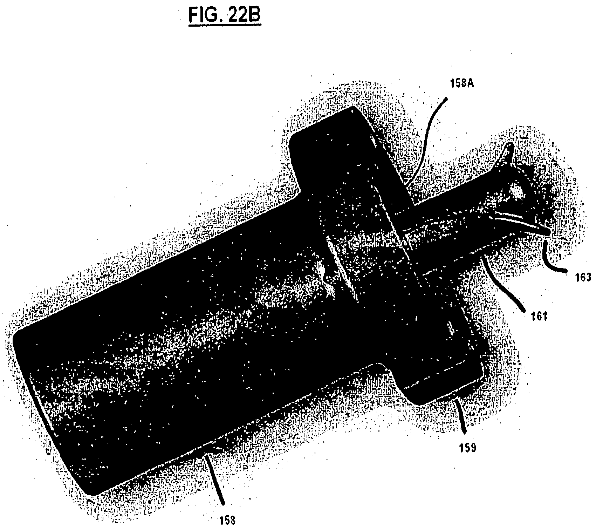

Referring to Figs. 22, 22A and 22B, there is depicted yet another embodiment of a downstream flow straightener according to the present invention. The flow straightener has a straight cylinder tube 158 having a flow chamber 158A and a base 159. A blade carrying center post 161 extends axially through at least a portion of the flow chamber 158A and extends axially into the pump chamber of an associated axial flow pump. In one embodiment, the center post 161 is affixed to the inner sidewall of the flow chamber 158A at a point of connection 162. (Fig. 22A). The support for the centerpost 162 may be a radially extending connecting arm 161A that need not traverse the entire diameter of the flow chamber. The post 161 may be of any shape, but is here depicted as a dome-topped cylinder having a pair of symmetrical diametrically opposed, contoured and pointed blade sections 163 extending longitudinally along at least part of its length and beyond by a predetermined amount. The blade sections 163 may protrude like rabbit ears beyond the top of the center post and may curve in opposite directions away from the axis of the center post 161 as depicted in Fig. 22B. The purpose of this configuration is also to diminish the rotational momentum of the axial flow output from the pump.

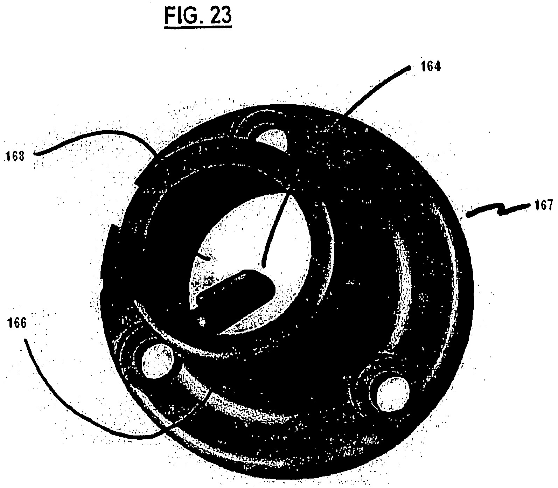

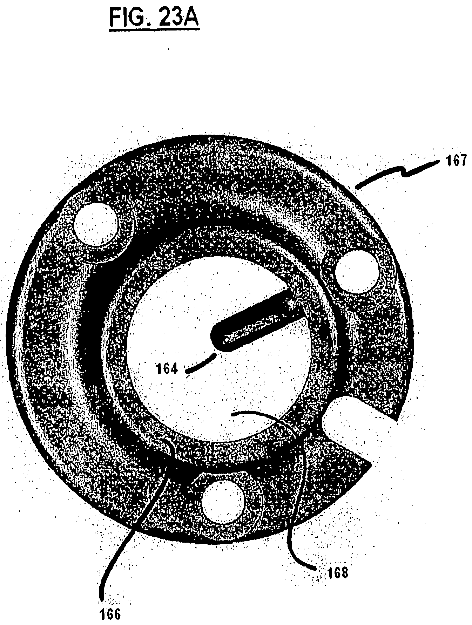

Referring to Figs. 23 and 23A, there is depicted a downstream flow straightener with a simplified flow-straightening blade element 164 according to another embodiment of the present invention. In this embodiment, a straight cylindrical tube section 166 is connected to a base 167. The tube 166 is affixed at the output from an axial flow pump and defines an internal flow chamber 168. The flow-straightening blade element 164 projects radially inwardly from an inner sidewall of the flow chamber 168. The blade element 164 may be suitably welded to the sidewall of the flow chamber or be formed together with and as part of the tube section 166. The blade element 164 is configured with a transverse axis aligned parallel to or co-axial with the axis of the flow chamber 168. In one embodiment the blade element 164 terminates at about the longitudinal centerline of the flow chamber 168 (Fig. 23A). It may, however, traverse completely across the flow chamber along a diameter (not shown) without departing from the scope of the present invention. The axis of the longitudinal tube section 166 is co-axial with the axis of the axial flow pump. The blade 164 may be substantially wedge-shaped and may be short (as shown) or longer. For example, the blade 164 may extend axially through the entire length of the tube 166 and may even extend beyond the length of the tube 166, as desired. According to an embodiment of the present invention, two rotary pumps as described herein may be combined to form an artificial heart that may be used to completely replace the natural heart in a patient suffering from heart failure.

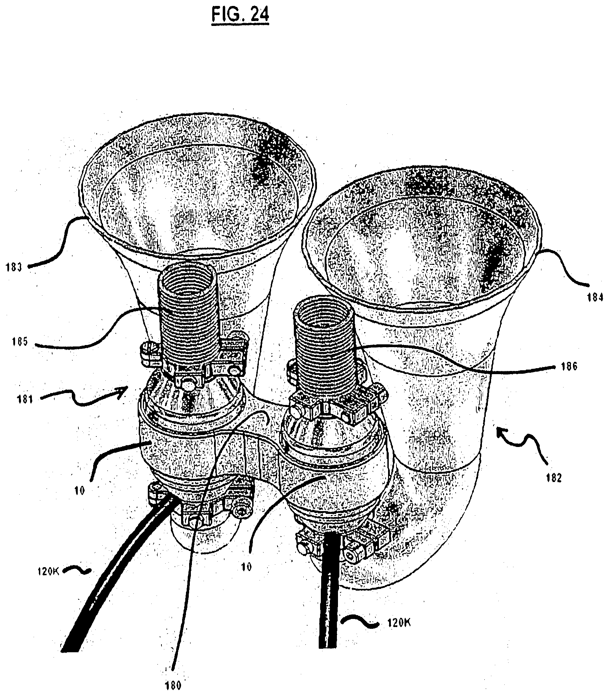

Referring to Fig. 24, an artificial heart is shown using rotary axial flow blood pumps of the type described herein. In one embodiment, the artificial heart may comprise a first section 181 for pumping blood to the patient's aorta and a second section 182 for pumping blood to the patient's pulmonary artery. Each section 181 and 182 may contain a pump 10 as described in detail above. The first section 181 may include a first inflow 183 and a first outflow 185. The second section 182 may include a second inflow 184 and a second outflow 186.

The inflows 183 and 184 may be made of a penetrable material such as a soft Dacron texture material so that it may be easily sutured to the patient's circulatory system. The inflows 183 and 184 may have a shape that is wider at the end that is connected to the patient's circulatory system than at the end that is connected to the pump 10. The inflows 183 and 184 maybe elbowed so that the inflows 183 and 184 maybe proximal to the outflows 185 and 186.

The first pump section 181 and the second pump section 182 may be attached together by a connecting member 180 such as a bracket or the like.

In this embodiment, the artificial heart does not require artificial vales thereby improving device reliability.

A balance member or atrial shunt or shunt may optionally be connected between the first and second inflows 183 and 184 to substantially equalize or balance the flow of blood through the first and second inflows 183 and 184. Thus, when the pressure in the first and second inflow members is unbalanced, blood may be shunted between the inflow members. The shunt may include two ends where one of the ends is connected to the first inflow 183 and the opposing end of the shunt is connected to the second inflow 184. The shunt 124 may be integrally formed with each of the inflows 183 and 184. The shunt may automatically equalize or balance the hydraulic blood flow through each of the first and second sections 181 and 182. The shunt may therefore prevent one side of the artificial heart from over pumping the other side of the heart

The first section 181 may be designed to pump more blood than the section 184. According to one embodiment, the first section 183 is designed to pump 15% more blood than the second section 182.

Power and control cables 120K may be used to power and control each pump 10.

The above specific embodiments are illustrative, and many variations can be introduced on these embodiments without departing from the scope of the appended claims. For example, elements and/or features of different illustrative embodiments may be combined with each other and/or substituted for each other within the scope appended claims.

## Claims

1. A blood pump, comprising:
a pump housing having an inlet and an outlet;
a rotor within said pump housing for propelling the blood through the housing in a direction from the inlet to the outlet, said rotor having a rotational axis and a leading edge portion for engaging blood entering the housing at the inlet and a trailing edge portion for enhancing the discharge of blood, said rotor being provided with a plurality of spaced apart grooves each groove having a respective fluid entry channel at said leading edge portion and an axially directed fluid exit channel at said trailing edge portion, each of said grooves being defined by sidewall surfaces extending radially to a surface of the rotor so as to define respective arcuate peripheral land areas therebetween on the surface of the rotor, each said fluid exit channel being wider than at the respective fluid entry channel, said grooves having portions curved relative to said rotational axis to direct blood flow axially therein to enhance the discharge flow characteristics when said rotor is rotating; and
said peripheral land areas being magnetically coupled to a motor stator to enable said rotor to be rotated in a predetermined direction.

2. The blood pump of claim 1 in which said peripheral land surfaces comprise hydrodynamic thrust bearing surfaces.

3. The blood pump of claim 2 in which said bearing surfaces comprise shroud sidewalls extending substantially transverse to the rotational axis of the rotor;
and/or
comprising at least one magnetic bearing formed between said rotor and said pump housing to position said rotor.

4. The blood pump of claim 1 in which said rotor comprises one or more permanent magnetic poles in said peripheral land areas, the motor being of radial flux gap design, whereby axial, magnetic forces hold the rotor in position.

5. The blood pump of claim 1 in which the motor comprises a hermetically sealed tubular stator that slides over said pump housing and is secured thereto;
and/or
in which said pump housing is cylindrical; and/or
in which said rotor comprises at least one permanent magnet mounted therein; and/or
in which said pump housing is made from biocompatible titanium or ceramic; and/or
which comprises a plurality of said rotors axially spaced apart within said pump housing, each of said rotors having one of said motor stators to cause said rotors to rotate.

6. The blood pump of claim 1 in which said rotor has a conformal polymer coating defining a hermetic seal to prevent oxidation.

7. The rotor of claim 1 in which each of said flow channels has a central section substantially narrower than the fluid entry channel and the fluid exit channel.

8. The rotor of claim 1 in which said rotor has a cylindrical body and each of said peripheral land areas comprises a substantially arcuate thrust bearing surface having a tapered portion extending from a low point at an edge of one of said peripheral land areas to a high point on the periphery of said cylindrical body.

9. The rotor of claim 8 in which the axis of curvature of said tapered thrust bearing surface is not coaxial with the rotational axis;
and/or
in which said thrust bearing surfaces are defined on each side by a sidewall shroud extending from a high point at one of said land area surface edges to merge into the periphery of said cylindrical body.

10. The rotor of claim 1 in which the rotor is provided with a plurality of bores therein to provide dynamic balance during rotation thereof;
and/or
in which each of said grooves is of non-uniform depth along its length from said leading edge portion to said trailing edge portion; and/or
in which the rotor comprises isotropic or anisotropic material.

11. The blood pump of claim 1 in which said pump housing comprises a material adapted to minimize eddy current losses when said rotor is rotated by said motor stator.

12. The blood pump of claim 1 comprising a heat treated biomedical alloy, preferably in which said alloy consists of platinum and cobalt.

13. The blood pump of claim 1 in which one of said leading and trailing edge portions of said rotor is provided with axially retaining magnetic poles of one polarity.

14. The blood pump of claim 1 in which the blood pump comprises a plurality of said rotors positioned in axially spaced-apart alignment within said pump housing.

15. The blood pump of claim 14 in which said rotors are interconnected within said housing to rotate together in the same direction on a common shaft;
or
in which each rotor is suspended to rotate independently within said housing without a common shaft;
and/or
in which each of said rotors comprises a plurality of drive magnets and a motor comprised of said rotors comprises a radial flux gap design whereby magnetic forces suspend each rotor in axial position within said housing.

## Patentansprüche

1. Blutpumpe, umfassend:
ein Pumpengehäuse mit einem Einlass und einem Auslass;
einen Rotor innerhalb des Pumpengehäuses zum Treiben des Blutes durch das Gehäuse in eine Richtung vom Einlass zum Auslass, wobei der Rotor eine Rotationsachse und einen Vorderkantenabschnitt zum Aufnehmen von am Einlass in das Gehäuse eintretendem Blut und einen Hinterkantenabschnitt zum Fördern der Entladung von Blut aufweist, wobei der Rotor mit einer Vielzahl von voneinander beabstandeten Nuten bereitgestellt ist, wobei jede Nut einen entsprechenden Fluideintrittskanal an dem Vorderkantenabschnitt und einen axial geführten Fluidaustrittskanal an dem Hinterkantenabschnitt aufweist, wobei jede Nut durch Seitenwandoberflächen definiert ist, die sich radial zu einer Oberfläche des Rotors erstrecken, so dass sie dazwischen auf der Oberfläche des Rotors entsprechende bogenförmige periphere Stegbereiche definieren, wobei jeder Fluid-Austrittskanal breiter ist als der entsprechende Fluideintrittskanal, wobei die Nuten in Bezug auf die Rotationsachsen gekrümmte Abschnitte aufweisen, um Blutfluss axial darin zu leiten, um die Entladungsfließeigenschaften zu verbessern, wenn der Rotor rotiert; und
wobei die peripheren Stegbereiche magnetisch an einen Motor-Stator gekoppelt sind, um es dem Rotor zu ermöglichen, in eine vorbestimmte Richtung rotiert zu werden.

2. Blutpumpe nach Anspruch 1, wobei die peripheren Stegoberflächen hydrodynamische Drucklageroberflächen umfassen.

3. Blutpumpe nach Anspruch 2, wobei die Lageroberflächen Ummantelungsseitenwände aufweisen, die sich im Wesentlichen quer zur Rotationsachse des Rotors erstrecken; und/oder
umfassend wenigstens ein zwischen dem Rotor und dem Pumpengehäuse gebildetes magnetisches Lager, um den Rotor zu positionieren.

4. Blutpumpe nach Anspruch 1, wobei der Rotor einen oder mehrere permanentmagnetische Pole in den peripheren Stegbereichen umfasst, wobei der Motor eine radiale Flusslückenkonstruktion aufweist, wobei axiale, magnetische Kräfte den Rotor in Position halten.

5. Blutpumpe nach Anspruch 1, wobei der Motor einen hermetisch verschlossenen röhrenförmigen Stator umfasst, der über das Pumpengehäuse gleitet und daran gesichert ist; und/oder
wobei das Pumpengehäuse zylindrisch ist; und/oder wobei der Rotor wenigstens einen darin eingebauten Permanentmagneten umfasst; und/oder
wobei das Pumpengehäuse aus biokompatiblem Titan oder Keramik besteht; und/oder
die eine Vielzahl der Rotoren umfasst, die innerhalb des Pumpengehäuses axial voneinander beabstandet sind, wobei jeder Rotor einen oder mehrere Motor-Statoren aufweist, um die Rotoren zum Rotieren zu bringen.

6. Blutpumpe nach Anspruch 1, wobei der Rotor eine winkeltreue Polymerbeschichtung aufweist, die eine hermetische Abdichtung definiert, um Oxidierung zu verhindern.

7. Rotor nach Anspruch 1, wobei jeder der Fließkanäle einen mittigen Bereich aufweist, der im Wesentlichen enger ist als der Fluideintrittskanal und der Fluidaustrittskanal.

8. Rotor nach Anspruch 1, wobei der Rotor einen zylindrischen Körper aufweist und jeder der peripheren Stegbereiche eine im Wesentlichen bogenförmige Drucklageroberfläche mit einem verjüngten Abschnitt aufweist, der sich von einem tiefen Punkt an einer Kante von einem der peripheren Stegbereiche zu einem hohen Punkt der Peripherie des zylindrischen Körpers erstreckt.

9. Rotor nach Anspruch 8, wobei die Krümmungsachse der verjüngten Drucklageroberfläche nicht mit der Rotationsachse koaxial ist; und/oder
wobei die Drucklageroberflächen auf jeder Seite von einer Seitenwandummantelung definiert sind, die sich von einem hohen Punkt an einer der Stegbereichflächenkanten erstreckt, um in die Peripherie des zylindrischen Körpers überzugehen.

10. Rotor nach Anspruch 1, wobei der Rotor mit einer Vielzahl von Öffnungen darin bereitgestellt ist, um dynamisches Gleichgewicht während der Rotation davon bereitzustellen, und/oder
wobei jede der Nuten entlang ihrer Länge vom Vorderkantenabschnitt zum Hinterkantenabschnitt von einer nicht einheitlichen Tiefe ist; und/oder
wobei der Rotor isotropes oder anisotropes Material umfasst.

11. Blutpumpe nach Anspruch 1, wobei das Pumpengehäuse ein Material umfasst, das dafür ausgelegt ist, Wirbelstromverluste zu minimieren, wenn der Rotor von dem Motor-Stator rotiert wird.

12. Blutpumpe nach Anspruch 1, umfassend eine wärmebehandelte, biomedizinische Legierung, wobei die Legierung bevorzugt aus Platin und Kobalt besteht.

13. Blutpumpe nach Anspruch 1, wobei einer der Vorder- und Hinterkantenabschnitte des Rotors mit axial rückhaltenden magnetischen Polen mit einer Polarität versehen ist.

14. Blutpumpe nach Anspruch 1, wobei die Blutpumpe eine Vielzahl der Rotoren umfasst, die innerhalb des Pumpengehäuses in axial beabstandeter Ausrichtung positioniert sind.

15. Blutpumpe nach Anspruch 14, wobei die Rotoren innerhalb des Gehäuses untereinander verbunden sind, um zusammen auf einem gemeinsamen Schaft in die gleiche Richtung zu rotieren; oder
wobei jeder Rotor ausgesetzt wird, um
innerhalb des Gehäuses ohne einen gemeinsamen Schaft unabhängig zu rotieren; und/oder
wobei jeder der Rotoren eine Vielzahl von Antriebsmagneten umfasst und ein aus den Rotoren bestehender Motor eine radiale Flusslückenkonstruktion umfasst, wobei magnetische Kräfte jeden Rotor innerhalb des Gehäuses in axialer Position aussetzen.

## Revendications

1. Pompe sanguine, comprenant :
un carter de pompe ayant une entrée et une sortie ;
un rotor à l'intérieur dudit carter de pompe pour propulser le sang à travers le carter dans une direction allant de l'entrée à la sortie, ledit rotor ayant un axe de rotation et une partie de bord d'attaque pour engager le sang entrant dans le carter à l'entrée et une partie de bord de fuite pour améliorer la décharge de sang, ledit rotor étant muni d'une pluralité de rainures espacées, chaque rainure ayant un canal d'entrée de fluide respectif au niveau de ladite partie de bord d'attaque et un canal de sortie de fluide dirigé axialement au niveau de ladite partie de bord de fuite, chacune desdites rainures étant définie par des surfaces de paroi latérale s'étendant radialement vers une surface du rotor de manière à définir des superficies périphériques arquées respectives entre celles-ci sur la surface du rotor, chaque canal de sortie de fluide étant plus large qu'au niveau du canal d'entrée de fluide respectif, lesdites rainures ayant des parties courbées par rapport audit axe de rotation pour diriger l'écoulement de sang axialement dans celui-ci pour améliorer les caractéristiques d'écoulement de décharge lorsque ledit rotor est en rotation ; et
lesdites superficies périphériques étant couplées magnétiquement à un stator de moteur pour permettre audit rotor d'être mis en rotation dans une direction prédéterminée.

2. Pompe sanguine selon la revendication 1, dans laquelle lesdites superficies périphériques comprennent des surfaces d'appui de poussée hydrodynamiques.

3. Pompe sanguine selon la revendication 2, dans laquelle lesdites surfaces d'appui comprennent des parois latérales d'enveloppe s'étendant sensiblement transversalement à l'axe de rotation du rotor ; et/ou
comprenant au moins un appareil d'appui magnétique formé entre ledit rotor et ledit carter de pompe pour positionner ledit rotor.

4. Pompe sanguine selon la revendication 1, dans laquelle ledit rotor comporte un ou plusieurs pôles magnétiques permanents dans lesdites superficies périphériques, le moteur étant de conception à vide de flux radial, de sorte que les forces magnétiques axiales maintiennent le rotor en position.

5. Pompe sanguine selon la revendication 1, dans laquelle le moteur comprend un stator tubulaire hermétiquement scellé qui glisse sur ledit carter de pompe et est fixé à celui-ci ; et/ou
dans laquelle ledit carter de pompe est cylindrique ; et/ou dans laquelle ledit rotor comprend au moins un aimant permanent monté à l'intérieur ; et/ou
dans laquelle ledit carter de pompe est réalisé en titane ou en céramique biocompatible ;et/ou
qui comprend une pluralité desdits rotors espacés axialement à l'intérieur dudit carter de pompe, chacun desdits rotors présentant l'un desdits stators de moteur pour entraîner lesdits rotors en rotation.

6. Pompe sanguine selon la revendication 1, dans laquelle ledit rotor possède un revêtement de polymère conforme définissant un joint hermétique pour empêcher l'oxydation.

7. Rotor selon la revendication 1, dans lequel chacun desdits canaux d'écoulement présente une section centrale sensiblement plus étroite que celle du canal d'entrée de fluide et du canal de sortie de fluide.

8. Rotor selon la revendication 1, dans lequel ledit rotor possède un corps cylindrique, et chacune desdites superficies périphériques comprend une surface d'appui de poussée sensiblement arquée comportant une partie conique allant d'un point bas à un bord de l'une desdites superficies périphériques à un haut élevé sur la périphérie dudit corps cylindrique.

9. Rotor selon la revendication 8, dans lequel l'axe de courbure de ladite surface d'appui conique de poussée n'est pas coaxiale avec l'axe de rotation ; et/ou
dans lequel lesdites surfaces d'appui de poussée sont définies de chaque côté par une enveloppe de paroi latérale s'étendant à partir d'un point haut à l'un desdits bords de superficie pour fusionner dans la périphérie dudit corps cylindrique.

10. Rotor selon la revendication 1, dans lequel le rotor est muni d'une pluralité de trous dans celui-ci pour fournir un équilibre dynamique pendant la rotation de celui-ci ; et/ou
dans lequel chacune desdites rainures a une profondeur non uniforme le long de sa longueur à partir de ladite partie de bord d'attaque vers ladite partie de bord de fuite ; et/ou
dans lequel le rotor comprend un matériau isotrope ou anisotrope.

11. Pompe sanguine selon la revendication 1, dans lequel ledit carter de pompe comprend un matériau adapté pour minimiser les pertes de courant de Foucault lorsque ledit rotor est mis en rotation par ledit stator du moteur.

12. Pompe sanguine selon la revendication 1, comprenant un alliage biomédical traité thermiquement, de préférence dans laquelle ledit alliage est constitué de platine et de cobalt.

13. Pompe sanguine selon la revendication 1, dans laquelle l'une desdites parties d'attaque et de fuite de bord dudit rotor est pourvue de pôles magnétiques, retenue axiale, d'une seule polarité.

14. Pompe sanguine selon la revendication 1, dans laquelle la pompe sanguine comprend une pluralité desdits rotors disposés dans un alignement axialement espacé à l'intérieur dudit carter de pompe.

15. Pompe sanguine selon la revendication 14, dans laquelle lesdits rotors sont reliés entre eux à l'intérieur dudit carter pour tourner ensemble dans la même direction sur un arbre commun ;ou
dans lequel chaque rotor est suspendu pour tourner indépendamment
à l'intérieur dudit carter sans arbre commun ; et/ou
dans lequel chacun desdits rotors comprend une pluralité d'aimants d'entraînement et un moteur constitué desdits rotors comprend une conception à vide de flux radial, de sorte que des forces magnétiques suspendent chaque rotor en position axiale à l'intérieur dudit carter.
